(19) **Europäisches Patentamt / European Patent Office / Office européen des brevets**

(11) **EP 2 341 839 B1**

(12) **EUROPEAN PATENT SPECIFICATION**

(45) Date of publication and mention of the grant of the patent:
**21.10.2015 Bulletin 2015/43**

(21) Application number: **09815350.5**

(22) Date of filing: **21.09.2009**

(51) Int Cl.:
*A61B 17/00* (2006.01)

(86) International application number:
**PCT/US2009/057728**

(87) International publication number:
**WO 2010/033940 (25.03.2010 Gazette 2010/12)**

(54) **System for vascular ultrasound treatments**

System zur Ultraschallbehandlung von Blutgefässen

Système de traitements vasculaires par ultrasons

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.09.2008 US 99155 P**

(43) Date of publication of application:
**13.07.2011 Bulletin 2011/28**

(73) Proprietor: **Vessix Vascular, Inc.**
**Laguna Hills, CA 92653 (US)**

(72) Inventors:
• **GUSTUS, Rolfe Tyson**
  **Laguna Hills, CA 92653 (US)**
• **STONE, Corbett W.**
  **Laguna Hills, CA 92653 (US)**
• **HOEY, Michael F.**
  **Laguna Hills, CA 92653 (US)**
• **BLANCK, Arthur G.**
  **Laguna Hills, CA 92653 (US)**
• **BRIGGS, Len**
  **Laguna Hills, CA 92653 (US)**
• **PERRY, Mike**
  **Laguna Hills, CA 92653 (US)**
• **MAZOR, Meital**
  **Laguna Hills, CA 92653 (US)**
• **KUNSTMANAS, Linas R.**
  **Laguna Hills, CA 92653 (US)**

(74) Representative: **Vossius & Partner**
**Patentanwälte Rechtsanwälte mbB**
**Siebertstrasse 3**
**81675 München (DE)**

(56) References cited:
**WO-A2-2008/126070       US-A- 6 033 397**
**US-A1- 2002 010 502      US-A1- 2003 069 619**
**US-A1- 2003 153 905      US-A1- 2004 215 296**
**US-A1- 2004 220 556      US-A1- 2005 251 116**
**US-A1- 2005 251 116      US-A1- 2007 077 230**
**US-A1- 2007 239 062      US-A1- 2008 125 772**
**US-A1- 2008 125 772      US-A1- 2008 188 913**
**US-B2- 6 599 288**

• **HUTCHINSON E B ET AL: "MRI feedback control for phased array prostate hyperthermia", ULTRASONICS SYMPOSIUM, 1996. PROCEEDINGS., 1996 IEEE SAN ANTONIO, TX, USA 3-6 NOV. 1996, IEEE, NEW YORK, NY, USA, vol. 2, 3 November 1996 (1996-11-03), pages 1285-1288, XP010217672, DOI: 10.1109/ULTSYM. 1996.584232 ISBN: 978-0-7803-3615-5**

**Description**

BACKGROUND OF THE INVENTION

The present disclosure is generally related to medical devices, systems, and methods, inter alia treatment for luminal diseases, particularly for atherosclerotic plaque, vulnerable or "hot" plaque, and the like. The structures of the invention allow remodeling artery tissue using gentle heat without ablation.

Balloon angioplasty and other catheters often are used to open arteries that have been narrowed due to atherosclerotic disease. The trauma associated with balloon dilation can impose significant injury, so that the benefits of balloon dilation may be limited in time. Stents are commonly used to extend the beneficial opening of the blood vessel. Restenosis or a subsequent narrowing of the body lumen after stenting has occurred in a significant number of cases.

More recently, drug coated stents (such as Johnson and Johnson's Cypher™ stent, the associated drug comprising Sirolimus™) have demonstrated a markedly reduced restenosis rate, and others are developing and commercializing alternative drug eluting stents. In addition, work has also been initiated with systemic drug delivery (intravenous or oral) which may also improve the procedural angioplasty success rates.

While drug eluting stents appear to offer significant promise for treatment of atherosclerosis in many patients, there remain many cases where stents either cannot be used or present significant disadvantages. Generally, stenting leaves an implant in the body. Such implants can present risks, including mechanical fatigue, corrosion, and the like, particularly when removal of the implant is difficult and involves invasive surgery. Stenting may have additional disadvantages for treating diffuse artery disease, for treating bifurcations, for treating areas of the body susceptible to crush, and for treating arteries subject to torsion, elongation, and shortening.

A variety of modified restenosis treatments or restenosis-inhibiting treatment modalities have also been proposed, including intravascular radiation, cryogenic treatments, ultrasound energy, and the like, often in combination with balloon angioplasty and/or stenting. While these and different approaches show varying degrees of promise for decreasing the subsequent degradation in blood flow following angioplasty and stenting, the trauma initially imposed on the tissues by angioplasty remains problematic.

A number of alternatives to stenting and balloon angioplasty so as to open stenosed arteries have also been proposed. For example, a wide variety of atherectomy devices and techniques have been disclosed and attempted. Despite the disadvantages and limitations of angioplasty and stenting, atherectomy has not gained the widespread use and success rates of dilation-based approaches. More recently, still further disadvantages of dilation have come to light. These include the existence of vulnerable plaque, which can rupture and release materials that may cause myocardial infarction or heart attack.

A system for ultrasonic ablation in body lumens is disclosed in US 6,599,288 B2.

In light of the above, it would be advantageous to provide systems for inducing vasodilation in artery tissue and remodeling of the lumens of the body. It would further be desirable to avoid significant cost or complexity while providing structures which could remodel body lumens without having to resort to the trauma of extreme dilation, and to allow the opening of blood vessels and other body lumens which are not suitable for stenting.

BRIEF SUMMARY OF THE INVENTION

The present invention is defined in the appended set of claims. The desirable temperature effects include mildly heating the tissue for treating atherosclerotic lesions and other disease states. While also being well-suited for treatment of occlusive diseases, the techniques of the present invention are particularly advantageous for treatment of patients who have (or are at risk of having) vulnerable plaques, regardless of whether those vulnerable plaques cause significant occlusion of an associated vessel lumen. Catheter systems of the present invention can incorporate optical coherence tomography or other imaging techniques which allow a structure and location of the diseased tissue to be characterized.

In a first aspect, the invention comprises a system for inducing desirable temperature effects on body tissue disposed about a lumen. The system includes an elongate catheter having a proximal end and a distal end with an axis therebetween with an energy delivery portion for transmission of energy. A tissue analyzer configured to characterize the body tissue in the lumen proximate the energy delivery portion and an energy source coupled to the energy delivery portion transmitting focused ultrasound energy. A processor is coupled to the tissue analyzer and to the energy source, wherein the processor is configured to determine an appropriate treatment energy for the characterized body tissue so as to mildly heat the body tissue with the energy delivery portion without ablating.

The system may be employed in a method for inducing desirable temperature effects on body tissue within a body lumen. The method includes positioning an energy delivery portion of a catheter within the lumen adjacent the tissue to be heated, and characterizing the tissue in the lumen proximate the energy delivery portion using a tissue analyzer. Then an appropriate treatment energy for the characterized tissue is determined using a processor coupled to the tissue analyzer. Then the energy delivery portion is energized with appropriate treatment energy from an energy source coupled to the processor. The tissue is mildly heated with the appropriate treatment energy, without ablating and without causing excessive thermal damage to the tissue, so as to induce a long-term occlusive response.

Preferably, the energy delivery portion comprises at least one radially oriented window coupled to at least one optical conduit extending between the proximal end of the catheter and the at least one window for transmission of laser energy to the body tissue from the laser energy source.

In many embodiments, the energy delivery portion comprises at least one ultrasound transducer configured to deliver ultrasound energy to the body tissue. The frequency of the energy is between 150 kHz and 5 MHz.

In many embodiments, the processor has predetermined treatment energy characteristics suitable for mildly heating different characterized materials.

In many embodiments, the processor is configured to adjust the treatment energy in response to feedback from the tissue analyzer during heating of the body tissue.

In many embodiments, the tissue analyzer comprises an optical coherence tomographer coupled to at least one optical conduit extending between the proximal end of the catheter and at least one radially oriented window. The tomographer generates image signals from imaging light from the body tissue so as to characterize the body tissue, the imaging light being transmitted through the at least one window.

In another aspect, the invention comprises a system for non-invasively inducing desirable temperature effects on tissue in a tissue treatment area within a body lumen. The system includes a focused ultrasound energy device configured to deliver focused ultrasound energy to the tissue treatment area, a tissue analyzer configured to characterize the tissue in the tissue treatment area, and a processor coupled to the tissue analyzer and focused ultrasound energy device, The processor is configured to determine appropriate focused ultrasound parameters for the characterized tissue so as to mildly heat the tissue with the appropriate focused ultrasound energy, without ablating, and without causing excessive thermal damage to the tissue, so as to induce a long-term occlusive response.

The system may be employed in a method for non-invasively inducing desirable temperature effects on tissue in a tissue treatment area within a body lumen. The method includes positioning a focused ultrasound energy device configured to deliver focused ultrasound energy to the tissue treatment area, characterizing the tissue in the lumen proximate the energy delivery portion using a tissue analyzer and determining an appropriate treatment energy for the characterized tissue using a processor coupled to the tissue analyzer and the focused ultrasound energy device. The focused ultrasound energy device is energized with appropriate treatment energy to mildly heat the tissue without ablating, and without causing excessive thermal damage to the tissue so as to induce a long-term occlusive response.

BRIEF DESCRIPTION OF THE DRAWINGS

FIG. 1 schematically illustrates one embodiment of a balloon catheter system for gently heating artery tissue.

FIG. 2 shows a plurality of ultrasonic transducers or microwave antennas mounted on a balloon surface for using in a catheter system.

FIG. 3 shows a plurality of transducers or antennas mounted on a stent-like cage for use with a catheter system.

FIG. 4 shows a cross-section view of a balloon and transducers inflated in an artery having plaque or lesion and calcium deposits.

FIG. 5 shows over-lapping wave patterns of ultrasonic energy when focusing transducers are used.

FIG. 6 shows a cross-section view of a balloon and transducers inflated in an artery with two different plaques being treated by the ultrasonic catheter.

FIG. 7 shows an alternative way to induce heat in an artery having plaque or lesion using an ultrasonic catheter that is depth/tissue specific for gentle heating.

FIG. 8 shows a pattern of waves emitted from a single unfocused transducer.

FIG. 9 shows a cross-section view of a balloon with a single unfocused transducer inflated in an artery emitting unfocused ultrasonic energy into plaque or lesion for gentle heating.

FIGs. 10A-10C show cross-section views of a balloon inflated in an artery transmitting ultrasonic energy toward plaque or lesion using an array of transducers, a pair of transducers, or a single transducer.

FIG. 11 shows a catheter with transducers mounted on the inside surface of the balloon.

FIGs. 12A and 12B show non-invasive treatment using external ultrasound to treat a diseased or plaque portion of an artery within a body.

FIG. 13A shows an approximation of two point sources separated by a distance "s" having a wavelength less than "s".

FIG. 13B shows an approximation of two point sources separated by a distance "s" having a wavelength greater than "s" for heating below a surface.

FIG. 14A shows an example of a minimum configuration with a spacing $S_{min}$ of 0.2 mm and depth $d_{min}$ of 0.1mm used in calculating a minimum frequency.

FIG. 14B shows an example of a maximum configuration with a spacing $S_{max}$ of 2.0 mm and depth $d_{max}$ of 5.0 mm used in calculating a maximum frequency.

FIG. 15 shows a laser based catheter system designed to gently heat body tissue using laser energy.

FIG. 16 shows a cross-section view of a distal end of the laser catheter of FIG. 15.

FIGs. 17A-17D show a laser catheter for gentle heating a body lumen.

## DETAILED DESCRIPTION OF THE INVENTION

Many therapies have been developed to replace or improve upon traditional balloon angioplasty and stents. The alternative devices described in the BACKGROUND OF THE INVENTION either cut, ablate, or vaporize diseased tissue in an artery. For example, laser devices vaporize plaque and flush it downstream. Atherectomy devices excise plaque and suck it out of the body. Cutting balloons incise the artery wall, damaging the tissue.

It would be advantageous to provide systems and devices that do not cut, ablate, or vaporize. Three modalities of treatment avoid these drawbacks and include: cooling the tissue; non-ablative forms of direct molecular denaturing; and non-ablative heating. Cooling has been implemented using devices such as Boston Scientific's Cryo-cath. Direct molecular denaturing can be achieved with radiation -- gamma rays, for instance.

The present invention is directed to the remaining modality, non-ablative heating using focused ultrasound energy. The embodiments disclosed herein revolve around the concept of "gentle heating" of diseased tissue in an artery, regardless of the specific treatment modality or technological implementation. The treatment of the diseased artery is achieved using a device inside the artery without ablation, while other examples disclose non-invasive treatment from outside the body using external devices.

While generally described herein with reference to the vasculature, embodiments of the catheter , systems described herein may also find applications in the lumens of other vessels of the human anatomy. The anatomical structure into which the catheter is placed may be for example, the esophagus, the oral cavity, the nasopharyngeal cavity, the auditory tube and tympanic cavity, the sinus of the brain, the larynx, the trachea, the bronchus, the stomach, the duodenum, the ileum, the colon, the rectum, the bladder, the ureter, the ejaculatory duct, the vas deferens, the urethra, the uterine cavity, the vaginal canal, and the cervical canal, as well as the arterial system, the venous system, and/or the heart

Remodeling involves the application of ultrasound energy. This energy is controlled so as to limit a temperature of target and/or collateral tissues, for example, limiting the heating of a fibrous cap of a vulnerable plaque or the intimal layer of an artery structure. In some embodiments, the surface temperature range for gentle heating is from about 45°C to about 99°C. For mild gentle heating, the surface temperature may range from about 45°C to about 65°C, while for more aggressive gentle heating, the surface temperature may range from about 65°C to about 99°C. Limiting heating of a lipid-rich pool of a vulnerable plaque sufficiently to induce melting of the lipid pool while inhibiting heating of other tissues (such as an intimal layer or fibrous cap) to less than a surface temperature in a range from about 50°C to about 65°C, such that the bulk tissue temperature remains mostly below 50°C - 55°C may inhibit an immune response that might otherwise lead to restenosis, or the like. Relatively mild temperatures between 50°C and 65°C may be sufficient to denature and break protein bonds during treatment, immediately after treatment, and/or more than one hour, more than one day, more than one week, or even more than one month after the treatment through a healing response of the tissue to the treatment so as to provide a bigger vessel lumen and improved blood flow.

The length of time or average rate of energy delivery for gently heating of body tissue may also vary. For example, in some embodiments the average rate of energy delivery to the tissue is on the same order of magnitude as the rate of energy dissipation by the tissue. In other embodiments, the delivered energy is sufficiently low that differences in tissue properties, including thermal conduction, heat capacity, innate blood perfusion, and distance from well perfused tissue, cause heat to be drawn from the healthy tissue at a rate that avoids significant thermal damage to the healthy tissue, while allowing heat to build up in diseased tissue,

The devices, systems and methods described herein are not selective in tissue treatment of the blood vessel and can be used for treatment of both concentric and eccentric atherosclerosis. This is a particular advantage because atherosclerosis may be eccentric relative to an axis of the blood vessel over 50% of the time, possibly in as much as (or even more than) 75% of cases.

Hence, remodeling of atherosclerotic materials may comprise shrinkage, melting, and the like of atherosclerotic and other plaques. Atherosclerotic material within the layers of an artery may be denatured, melted and/or the treatment may involve a shrinking of atherosclerotic materials within the artery layers so as to improve blood flow. The invention may also provide particular advantages for treatment of vulnerable plaques or blood vessels in which vulnerable plaque is a concern, which may comprise eccentric lesions. The invention will also find applications for mild heating of the cap structure (to induce toughening of the cap and make the plaque less vulnerable to rupture) and/or heating of the lipid-rich pool of the vulnerable plaque (so as to remodel, denature, melt, shrink, and/or redistribute the lipid-rich pool).

Catheter Based Treatment

Some embodiments of the present invention generally provide systems for inducing desirable temperature effects on artery tissue, particularly atherosclerotic diseased tissue, by gentle heating in combination with gentle or standard dilation. In many embodiments, the disclosed system comprises at least an energy generator and a catheter. The catheter may be similar to a balloon catheter commonly used to treat artery disease today, except for the addition of ultrasonic transducers . The system will be able to treat diseased tissue by gentle heating in combination with dilation of the artery. In case of calcification in the artery plaque, it may be more difficult to remodel and open the diseased artery, so the catheter may use a standard angioplasty balloon in combination with ultrasonic energy to break down the calcium and remodel and open the lumen.

In one embodiment, an angioplasty balloon catheter structure having transducers disposed thereon might apply ultrasound heating to the vessel wall. The heating of the vessel wall may be done before, during, and/or after dilation, optionally in combination with dilation pressures which are at or significantly lower than standard, unheated angioplasty dilation pressures. For example, where balloon inflation pressures of 10-16 atmospheres may be appropriate for standard angioplasty dilation of a particular lesion, modified dilation treatments combined with gentle heating described herein may employ from 10-16 atmospheres or may be effected with pressures of 6 atmospheres or less, and possibly as low as 1 to 2 atmospheres.

The gentle heating energy added before, during, and or after dilation of a blood vessel may increase dilation effectiveness while lowering complications. In some embodiments, such controlled heating with balloon dilatation may exhibit a reduction in recoil, providing at least some of the benefits of a stent-like expansion without the disadvantages of an implant. Benefits of gentle heating may be enhanced (and/or complications inhibited) by limiting heating of the adventitial layer below a deleterious response threshold.

While the present invention may be used in combination with stenting, the present invention is particularly well suited for increasing the open diameter of blood vessels in which stenting is not a viable option. Potential applications include treatment of diffuse disease, in which atherosclerosis is spread along a significant length of an artery rather than being localized in one area. The invention may also find advantageous use for treatment of tortuous, sharply-curved vessels, as no stent need be advanced into or expanded within the sharp bends of many blood vessel. Still further advantageous applications include treatment along bifurcations (where side branch blockage may be an issue) and in the peripheral extremities such as the legs, feet, and arms (where crushing and/or stent fracture failure may be problematic).

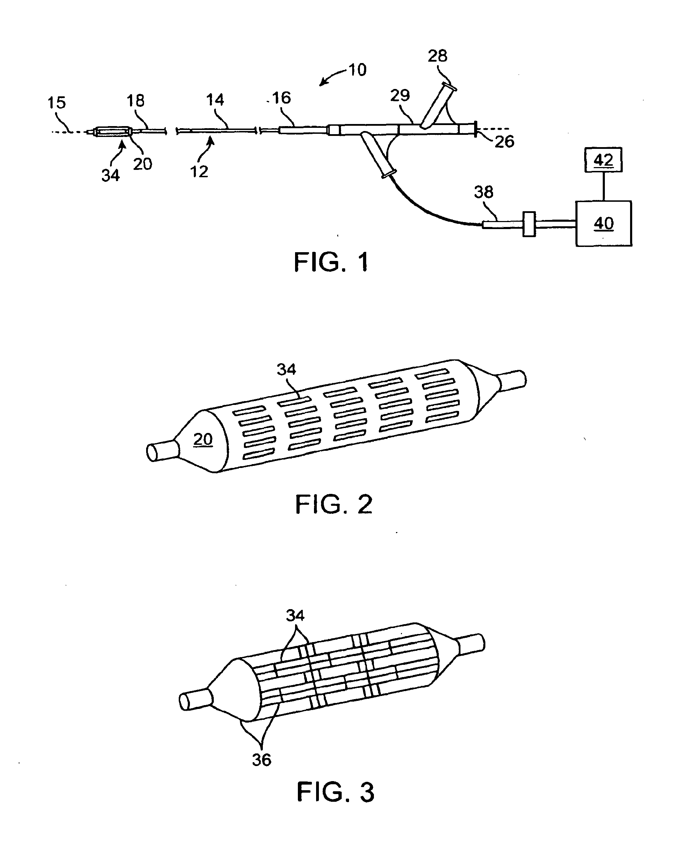

Fig. 1 shows one embodiment of a balloon catheter system 10 for inducing desirable temperature effects on artery tissue. The catheter system 10 includes a balloon catheter 12 having a catheter body 14 with a proximal end 16 and a distal end 18 with an axis 15 therebetween. Catheter body 14 is flexible and may include one or more lumens, such as a guidewire lumen and an inflation lumen. Still further lumens may be provided if desired for other treatments or applications, such as perfusion, fluid delivery, imaging, conductor lumen, or the like. Catheter 12 includes an inflatable balloon 20 adjacent distal end 18 and a housing 29 adjacent proximal end 16. Housing 29 includes a first connector 26 in communication with the guidewire lumen and a second connector 28 in fluid communication with the inflation lumen. The inflation lumen extends between balloon 20 and second connector 28. Both first and second connectors 26, 28 may optionally comprise a standard connector, such as a Luer-Loc™ connector. A distal tip may include an integral tip valve to allow passage of guidewires, and the like.

Ultrasound transducers 34 are mounted on a surface of balloon 20, covering the balloon partially or fully, with associated conductors extending proximally from the transducers . In some examples, the transducers or antennas 34 may be positioned internal of balloon 20. Transducers or antennas 34 may be arranged in many different patterns or arrays on balloon 20. In some embodiments, adjacent transducers are axially offset. In other embodiments, transducers may be arranged in bands around the balloon. The transducers are focusing transducers.

Housing 29 also accommodates an electrical connector 38. Connector 38 includes a plurality of electrical connections, each electrically coupled to the transducers 34 on the balloon surface via conductors, shown in FIG. 2. This allows the transducer 34 to be easily energized by a controller 40 and a power source 42 of ultrasound energy.

In some embodiments, the balloon may be made as an ultrasonic transducer with a transmitting layer comprising piezoelectric material, having a relatively high dielectric constant and a relatively high acoustic impedance. Upon inflation, the balloon, which is actually the ultrasonic transducer, will emit ultrasonic energy toward the plaque. This system will be able to treat athrosclerosis disease by gentle heating with gentle or standard dilation.

The ultrasonic transducer balloon can be built in different forms, such as:

1. The entire balloon is made of piezoelectric plastic.

2. Only part of the balloon is made of piezoelectric plastic, and thus can treat specifically diseased tissue, by rotating before inflation, and after deflation.

3. By using a receiving layer comprising piezoelectric material or any other suitable material, wherein the layers (transmitting and receiving) are interconnected in a laminar manner, the catheter may also be able to diagnose, e.g., IVUS catheter. This will enable detection of plaque depth and tissue type, during the treatment, using only one catheter.

4. Discrete subsets of the balloon surface material are embedded with ultrasonic transducer elements. Then, the discrete elements are independently connected to an external generator that can activate the elements independently and at independent power, frequency, etc., if desired. This independent activation allows for selective dosing within a lumen, as well as discretization of the treatment energy, which has other benefits. For instance, energy is more directed and evenly delivered rather than it flowing wherever the tissue composition and properties dictate, as is the case with non-selective, unfocused energy.

In other embodiments, the transducers 34 mentioned above can also be built in a form of a cage 36. For example, FIG. 3 shows an expandable cage built from transducers 34 mounted on a stent-like cage 36. This is possible with any method of creating apposition with a lumen wall. An advantage of a catheter built like this is that during treatment, the flow of fluid through the lumen doesn't stop. The stent-like cage with the transducers on it will be pulled out from the artery in the end of the treatment.

There is also the option in most embodiments of adding a transducer to the tip of the catheter in order to help with crossing a legion or blockage in the artery lumen.

Balloon 20 generally includes a proximal portion coupled to the inflation lumen and a distal portion coupled to the guidewire lumen. Balloon 20 expands radially when inflated with a fluid or a gas. In some embodiments, the fluid or gas may be non-conductive and/or cooled. In some embodiments, balloon 20 may be a low pressure balloon pressurized to contact the artery tissue. In other embodiments, balloon 20 is an angioplasty balloon capable of a higher pressure to expand the artery lumen while gentle heating is applied. Balloon 20 may comprise a compliant or non-compliant balloon having helical folds to facilitate reconfiguring the balloon from a radially expanded, inflated configuration to a low profile configuration, particularly for removal after use.

In some embodiments, the controller 40 may include a processor or may be coupled to a processor to control or record treatment. The processor will typically comprise computer hardware and/or software, often including one or more programmable processor-unit running, machine-readable program instructions or code for implementing the methods described herein. The code may be embodied in a tangible media such as a memory (optionally a read only memory, a random access memory, a non-volatile memory, or the like) and/or a recording media (such as a floppy disk, a hard drive, a CD, a DVD, a non-volatile solid-state memory card, or the like). The code and/or associated data and signals may also be transmitted to or from the processor via a network connection (such as a wireless network, an Ethernet, an Internet, an Intranet, or the like), and the code may also be transmitted between the components of the catheter system 10 and within a processor via bus, and appropriate standard or proprietary communications cards, connectors, cables, and the like. The processor may be configured to perform calculations and signal transmission steps described herein at least in part by programming the processor with the software code, which may be written as a single program, a series of separate subroutines or related programs, or the like. The processor may comprise standard or proprietary digital and/or analog signal processing hardware, software, and/or firmware, and will typically have sufficient processing power to perform the calculations described herein during treatment of the patient. The processor may relate to a personal computer, a notebook computer, a tablet computer, a proprietary processing unit, or a combination thereof. Standard or proprietary input devices (such as a mouse, keyboard, touchscreen, joystick, etc.) and output devices (such as a printer, speakers, display, etc.) associated with modem computer systems may also be included, and processors having a plurality of processing units (or even separate computers) may be employed in a wide range of centralized or distributed data processing architectures.

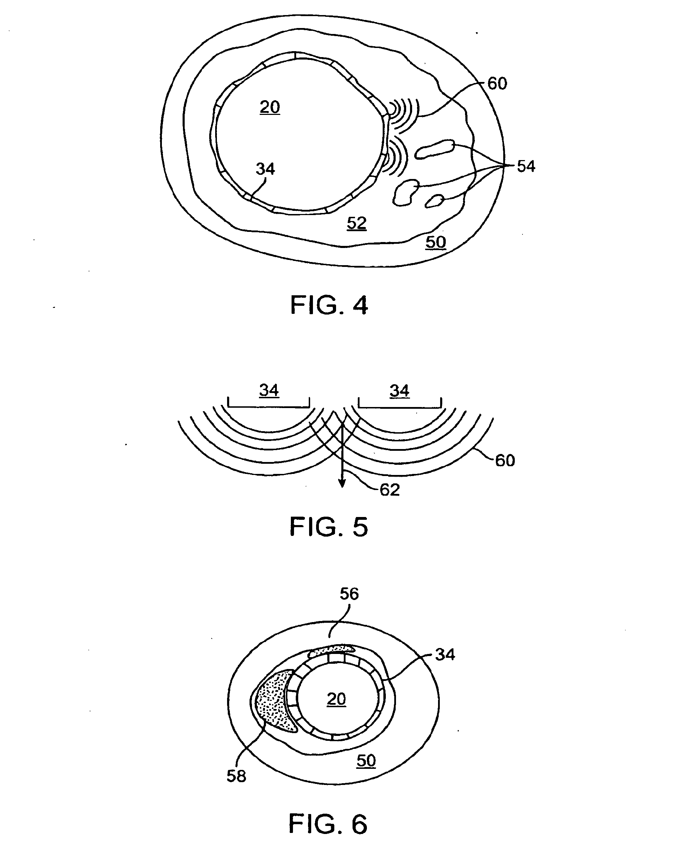

FIG. 4 shows a cross-section view of the balloon 20 and the transducers 34, inflated in an artery 50 having plaque or lesion 52 and calcium deposits 54. The transducers 34 emit ultrasonic energy 60 into a plaque or lesion 52. The ultrasonic energy 60 creates heat and mildly heats the plaque or lesion 52. The ultrasonic energy 60 may potentially break up the calcium deposits 54. The heat has numerous potential advantages, including collagen shrinkage and debulking. Breaking calcium is also an advantage over existing products. Note also that the native vessel or artery 50 helps to maintain pressure on the plaque or lesion 52, to keep it in contact with transducers 34.

FIG. 5 shows overlapping wave patterns 60 of ultrasonic energy when focusing transducers 34 are used. Arrow 62 represents the location of hotspots, which are locations where waves from different transducers combine, causing spots with a higher temperature. The arrow 62 represents the depth of the plaque. This system enables imposing constructive interference. Using different frequencies enables the system to modulate wavelength, thus focusing on different depth of the plaque. Considering that the plaque is not homogenous, this system is able to be "disease-area/location-specific" by modulating differences in a wavelength between the transducers.

FIG. 6 shows a cross-section view of the balloon 20 and the transducers 34 inflated in an artery 50 with two different plaques 56, 58 being treated by the ultrasonic catheter 10. The balloon 20 is covered partially or fully by the transducers 34. The little plaque 56 is treated with shorter waves because of its shallow depth. The deeper plaque 58 will be treated with longer waves because of its greater depth. As the ultrasonic power increases, the temperature inside the plaque gets higher.

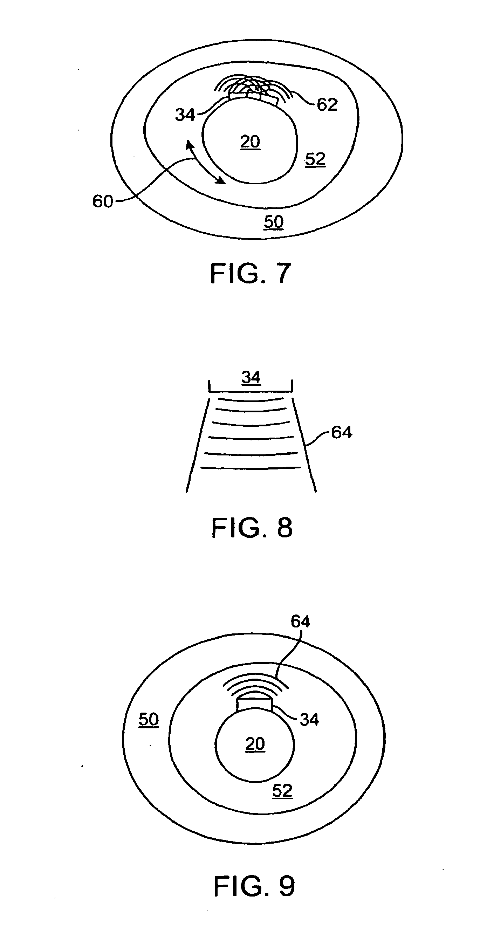

FIG. 7 shows an alternative way to induce heat in the artery 50 having plaque or lesion 52 using an ultrasonic catheter that is depth/tissue specific for gentle heating. In this case, the balloon 20 is only partially covered with the focusing transducers 34, but the balloon is capable of rotating 60 within the artery, thus applying different wavelength 62 at a time to specific treatment areas, depending on the depth of the plaque and on the tissue type. This method will also avoid treating healthy tissue.

FIG. 8 shows a pattern of waves 64 emitted from an unfocused single transducer 34. FIG. 9 shows a cross-section view of the balloon 20 with a single unfocused transducer 34, inflated in the artery 50, emitting unfocused ultrasonic energy 64 into the plaque or lesion 52 for gentle heating. Also in this embodiment, the power level may vary depending on the plaque depth and on the tissue type.

The transducers may be arranged on the surface of the balloon and inside the balloon. For example, a transducer wire/core may be positioned near a center of the balloon and be tuned to pass through saline and into tissue, preferably targeting calcium or thrombus.

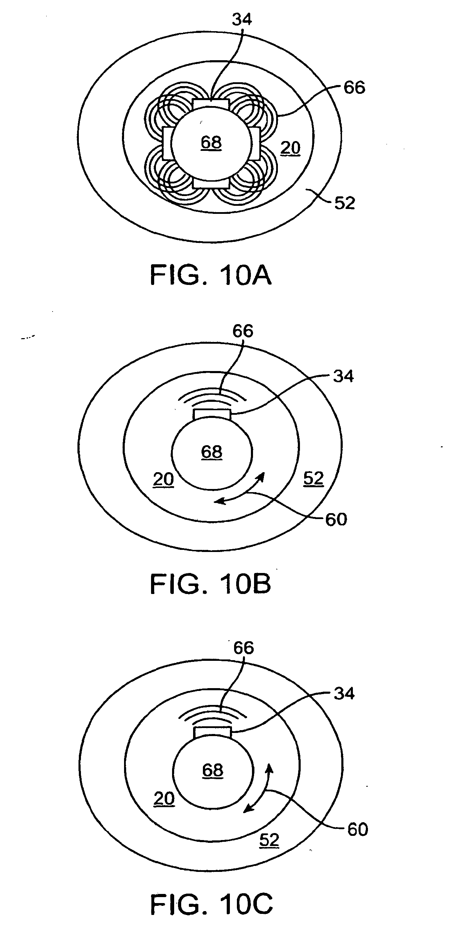

FIGs. 10A-10C show cross-section views of the balloon 20 inflated in the artery 50, transmitting ultrasonic energy 66 toward the plaque or lesion 52. In FIG. 10A, the whole array of the focusing transducers 34 is arranged on an inner core within the balloon 20. In FIG. 10B, a pair of focusing transducers 34 are on an inner core 68 located within the balloon 20 that can rotate 60. In FIG. 10C, an unfocused single transducer 34 is positioned on an inner core 68 within balloon 20 that can rotate 60. Fig. 10C does not form part of the present invention.

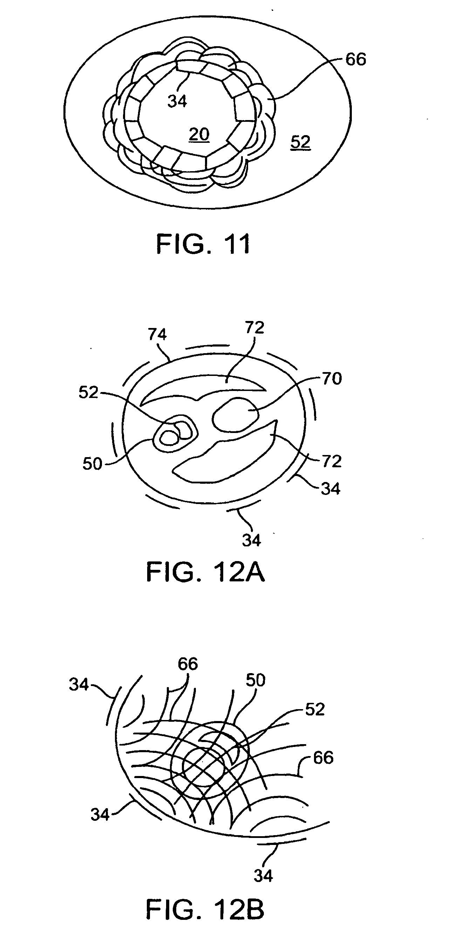

FIG. 11 shows a catheter with the transducers 34 mounted on the inside surface of the balloon 20. In some cases, the balloon can act as a lens or a diaphragm. In this case, the energy 66 doesn't have to pass through saline, hence there is less risk for delaminating transducers. This kind of catheter can also be built with one facet of focusing transducers on the inside surface of the balloon that can rotate after deflation. It also can be built with an unfocused single transducer on the inside surface of the balloon that can rotate.

A catheter may be built with transducers on the outside surface of the balloon and also inside the balloon, whether in the center or on the inside surface of the balloon.

The following examples, apart from examples concerning focused ultrasound energy, do not form part of the present invention.

External ultrasound

A further example relates to systems and methods of non-invasively treating diseased or unwanted tissue or substance inside a human or animal body. In particular, this example relates to image-guided external focused ultrasound used to treat atherosclerosis.

This example works as follows: If it is known where a plaque or diseased tissue is located, and if its topology (presumably from some kind of imaging device, such as MRI or IVUS, VH or otherwise) is known, then the plaque or diseased tissue can be targeted using a focused ultrasound treatment that is external to the body.

FIGs. 12A and 12B show an example using external ultrasound to treat a diseased or plaque portion 52 of an artery 50 within the body, for example, a leg 74 having muscles 72 and a bone 70. A plurality of ultrasonic transducers 76 is positioned around the leg 74, transmitting ultrasonic energy 78 toward the plaque or lesion 52. The ultrasonic energy 78 may potentially break up the calcium deposits. As the ultrasonic energy waves 78 from different transducers combine, spots with higher temperatures are created to mildly heat the plaque or lesion 52.

If the location of a plaque or diseased tissue is known from imaging, a control system (processor) can be used to activate discretely and selectively the ultrasonic transducers with independently specified parameters such as frequency, phase, power, etc., in order to target the diseased tissue as needed. This method has the advantage of being totally noninvasive and potentially a lot faster than traditional angioplasty.

Ultrasound Calculations Example

Calculations related to a depth of penetration and a minimum desired surface depth of treatment and its frequency dependence are discussed bellow. Making some assumptions about how the ultrasonic transducers might be disposed about the treatment catheter, e.g., on a balloon, it is possible to estimate the appropriate wavelengths and therefore frequencies that would be useful in implementing an ultrasonic catheter with focused waves.

The waves must constructively interfere in order to be maximally effective (create the hot spots). Ultrasound waves travel at different values of speed through different media. For these calculations, we assume that the sound travels through the tissue at approximately the same speed as through saline, roughly 1500 m/s.

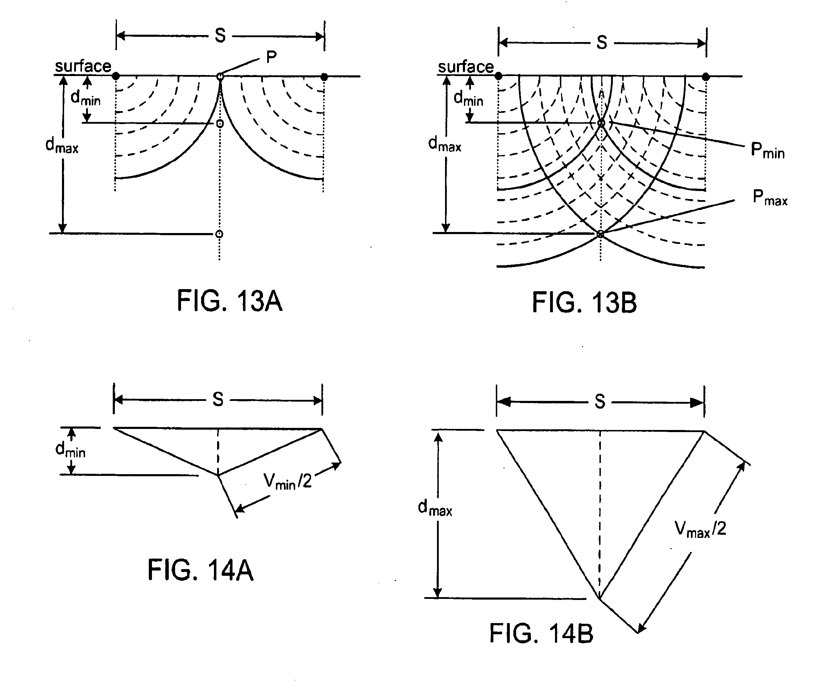

FIG. 13A shows an approximation of two point sources separated by a distance "s" having a wavelength less than "s". This is not a particularly useful implementation because the focus of the energy and the maximum heating is at the surface between the sources where the waves intersect P. It is more useful to select a wavelength that allows one to create constructive interference below the surface of whatever organ or body lumen contacting the transducers. FIG. 13B shows the desired treatment for heating at a minimum depth $d_{min}$ of 0.1 mm and a maximum depth $d_{max}$ of 5 mm into the lumen wall, with a spacing between the sources of at least 0.2 mm and at most 2.0 mm. One set of the wavelength range can be calculated using the formulas:

$$f_{min} = \frac{v}{\lambda_{min}} \quad \text{(a)}$$

$$\lambda = \frac{v}{f} \quad \text{(b)}$$

FIG. 14A shows a minimum configuration with a spacing $S_{min}$ of 0.2 mm and a depth $d_{min}$ of 0.1mm, the wavelength $\lambda_{min}$ may be calculated as follows:

$$\left(\frac{\lambda_{min}}{2}\right)^2 = d^2{}_{min} + \left(\frac{S_{min}}{2}\right)^2 \quad \text{(c)}$$

$$\lambda_{min} = 2\left[(0.1\ mm)^2 + \left(\frac{0.2\ mm}{2}\right)^2\right]^{\frac{1}{2}} \text{ or } 0.2828\ mm \quad \text{(d)}$$

Solving now for maximum frequency,

$$f_{max} = \frac{v}{\lambda_{min}} = \frac{1500\ m/s}{0.2828\ mm} = 5.3\ \text{MHz} \quad \text{(e)}$$

FIG. 14B shows a maximum configuration with a spacing $S_{max}$ of 2.0 mm and a depth $d_{max}$ of 5.0 mm, the wavelength $\lambda_{max}$ may be calculated as follows:

$$\left(\frac{\lambda_{max}}{2}\right)^2 = d^2{}_{max} + \left(\frac{S_{max}}{2}\right)^2 \quad \text{(f)}$$

$$\lambda_{max} = 2\left[(5.0 \ mm)^2 + \left(\frac{2.0 \ mm}{2}\right)^2\right]^{\frac{1}{2}} \text{ or } 10.2 \text{ mm} \quad \text{(g)}$$

Solving now for minimum frequency,

$$f_{min} = \frac{v}{\lambda_{max}} = \frac{1500 \ m/s}{10.2 \ mm} = 147 \text{ kHz} \quad \text{(h)}.$$

The calculations suggest that an appropriate range of frequencies useful for this example of treating an arterial disease from the transducers mounted on a balloon configured to be arranged in the artery would be between roughly 150 kHz to 5 MHz. This frequency range is appropriate using a focused ultrasound approach, and more specifically a bipolar, two-transducer implementation. If the target tissue is located at a smaller or greater depth, the frequency may range from 1 kHz to 20 MHz.

Laser based Treatment

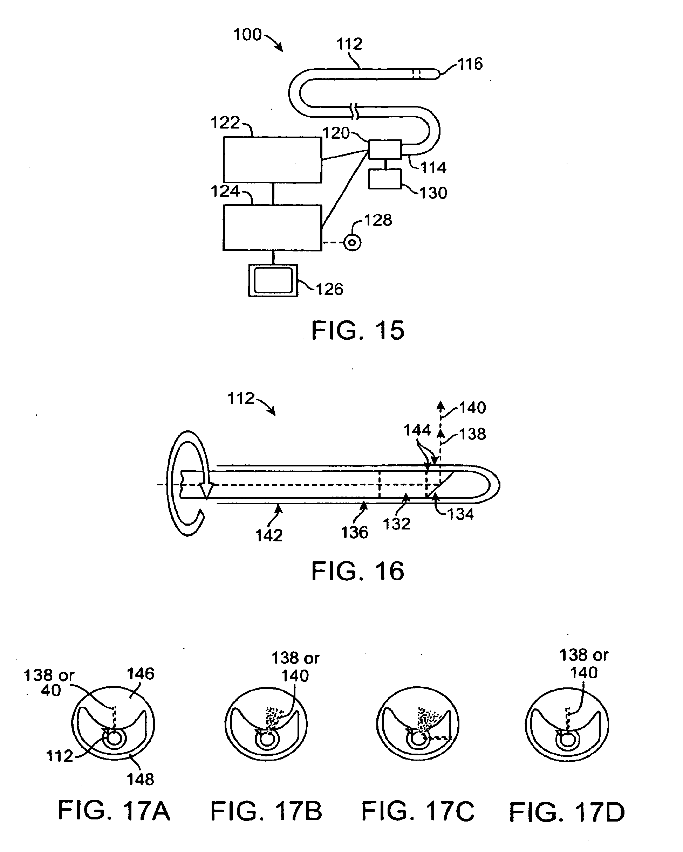

FIG. 15 shows a laser based catheter system 100 designed to gently heat body tissue using laser energy. A suitable system is disclosed in U.S. Patent Application No. 2005/0251116, filed May 3,2005, entitled "Imaging And Eccentric Atherosclerotic Material Laser Remodeling And/Or Ablation Catheter".
The catheter system 100 includes a catheter 112 having a proximal end 114 and a distal end 116 with an axis therebetween. A housing 120 adjacent proximal end 114 couples the catheter to a heating laser 122 and an analyzer 124, the analyzer often comprising an optical coherence tomography system. Optionally, a display 126 may show intravascular optical coherence tomography (or other) images, and may be used by a surgeon in an image-guided procedure. A drive 130 may effect scanning for at least one imaging component relative to a surrounding catheter sleeve, the scanning optionally comprising rotational scanning, helical scanning, axial scanning, and/or the like.
Additional system components, such as an input device for identifying tissues on the display for treatment and a processor for interpreting the imaging light signals from the catheter 112 may be incorporated into a laser or imaging system, or may be provided as stand-alone components. The analyzer 124 may include hardware and/or software for controlling the laser 122, the drive 130, the display 126, and/or the like. A wide variety of data processing and control architectures may be implemented, with the housing 120, the drive 130, the laser 122, the analyzer 124 and/or the display 126, optionally being integrated into one or more structures, into a number different housings, or the like. Machine-readable code with programming instructions for implementing some or all of the method steps described herein may be embodied in a tangible media 128, which may comprise a magnetic recording media, optical recording media, a memory such as a random access memory, read-only memory, or non-volatile memory, or the like. Alternatively, such code may be transmitted over a communication link such as an Ethernet, Internet, wireless network, or the like.
The catheter 112 gently heats body tissue using laser energy in any of a variety of wavelengths, often ranging from ultraviolet to infrared. This energy may be delivered from the laser 122 to a plaque or lesion by a fiber optic light conduit of the catheter 112. The laser 122 may comprise an excimer laser using ultraviolet light or optionally use electrically excited xenon and chloride gases. Laser 122 may be either a continuous wave or pulsed laser. Continuous wave lasers often lead to deep thermal penetration and may lead to possible charring and shallow craters, depending on the energy. A pulsed laser may reduce inadvertent heat conduction to surrounding tissues by providing sufficient time to permit thermal relaxation between laser pulses.
Referring now to FIGs. 16 and 17A-17D, the catheter 112 generally uses one or more bundles of one or more rotatable optical conduits (sometimes referred to as "optical probes") to direct light energy towards an artery wall at a given angle. The optical conduits may comprise one or more single-mode optical fiber, and may be housed inside a sleeve catheter or guidewire. The optical conduits may, at least in part, define optical paths, and each optical path may also be defined by a lens 132 and a fold mirror 134. The optical conduits may be used to convey light energy. The same optical conduit or bundle of optical conduits 136 may be used to convey the light energy for imaging, for instance imaging light 138, and the light energy for heating atherosclerotic plaques, e.g. remodeling light 140. The optical conduits 136 are housed inside a sleeve catheter or guidewire 142.

The optical conduits 136 rotate inside the sleeve catheter 142. The imaging light 138 runs through the optical conduits and radially through transparent cylindrical windows 142 to provide an intra-vascular image of the body lumen, for instance by OCT. The image is processed by a computer that identifies and localizes atherosclerotic plaques. Based on the information from the imaging, the computer then determines when to fire the heating light 140 such that the light gently heats the plaque 146 and does not damage the healthy area 148 of the artery. This may be done by pulsing light 140 on the plaque when the rotatable optical conduits face the plaque. The imaging and heating lights may be used sequentially.

Examples of the devices, system and methods described herein may be adjusted or tuned to provide the energy to gently heat the atherosclerotic materials. Characteristics of the energy, including the frequency, power, magnitude, delivery time, delivery location, and/or patterns or combinations thereof, may be predetermined before diagnosis or treatment of a specific patient, the energy characteristics being transmitted without feedback, such as by employing open-loop dosimetry techniques. Such predetermined characteristic tuning may be based on prior treatment of atherosclerotic materials, prior clinical trials, and/or other research. Energy may be tuned to be directed to a particular patient based on *in situ* feedback. Predetermined characteristics may also be used with others being feedback-controlled.

**Claims**

1. A system for mildly heating body tissue being disposed about a blood vessel, the system comprising:

   an elongate catheter having a proximal end and a distal end with an axis therebetween an inflatable balloon positioned adjacent to the distal end of the elongate catheter;
   an energy delivery portion configured to deliver focused ultrasound energy to the body tissue, said energy delivery portion including at least one ultrasound transducer mounted to an outer surface of the inflatable balloon;
   a tissue analyzer configured to characterize the body tissue in the blood vessel proximate the energy delivery portion;
   an energy source coupled to the energy delivery portion transmitting tissue treatment energy, wherein the energy source comprises ultrasound energy; and
   a processor coupled to the tissue analyzer and energy source, the processor configured to determine an appropriate treatment energy for the characterized body tissue by selecting an energy wavelength of the ultrasound energy based on characterization of the body tissue such that application of the appropriate treatment energy mildly heats the body tissue with the energy delivery portion without ablating the body tissue.

2. The system of claim 1, wherein the frequency of the energy is between 1 kHz to 20 MHz.

3. The system of claim 1, wherein at least one of:

   the processor has predetermined treatment energy characteristics suitable for mildly heating different characterized materials;
   the processor is configured to adjust the treatment energy in response to feedback from the tissue analyzer during heating of the body tissue.

4. The system of claim 1, wherein the tissue analyzer comprises an optical coherence tomographer coupled to at least one optical conduit extending between the proximal end of the catheter and at least one radially oriented window, the tomographer configured to generate image signals from imaging light from the body tissue so as to characterize the body tissue, the imaging light being transmitted through the at least one window.

5. The system according to claim 1 for mildly heating body tissue in proximity to a luminal surface having both healthy tissue and targeted tissue, wherein the processor is configured to determine an appropriate treatment energy to mildly heat the body tissue to a temperature in the range of from 45°C to 65°C, without ablating.

6. The system according to claim 1, wherein the processor is configured to determine appropriate focused ultrasound parameters for the characterized tissue so as to gently heat the tissue with the appropriate focused ultrasound energy to a temperature in the range of from 45°C to 65°C, without ablating.

7. The system of claim 6 for mildly heating tissue in proximity to a luminal surface comprising both healthy tissue and targeted tissue to a temperature in the range of from 45°C to 65°C sufficient to efficaciously alter the targeted tissue without causing excessive thermal damage to the healthy tissue.

8. The system of claim 6, wherein the tissue analyzer is a magnetic resonance imaging device.

9. The system of claim 6, wherein the processor is configured to adjust the focused ultrasound energy in response to feedback from the tissue analyzer during heating of the tissue.

10. The system of claim 6, wherein the processor has predetermined characteristics of focused ultrasound energy for gently heating different characterized tissues.


**Patentansprüche**

1. System zum sanften Erwärmen von Körpergewebe, das um ein Blutgefäß angeordnet ist, wobei das System aufweist:

einen länglichen Katheter, der ein proximales Ende und ein distales Ende mit einer Achse dazwischen aufweist;
einen aufblasbaren Ballon, der benachbart zum distalen Ende des länglichen Katheters angeordnet ist;
einen Energieverabreichungsabschnitt, der konfiguriert ist, fokussierte Ultraschallenergie an das Körpergewebe zu verabreichen, wobei der Energieverabreichungsabschnitt mindestens einen Ultraschallwandler aufweist, der an einer Außenfläche des aufblasbaren Ballons angebracht ist;
einen Gewebeanalysator, der konfiguriert ist, das Körpergewebe in dem zum Energieverabreichungsabschnitt nahegelegenen Blutgefäß zu charakterisieren;
eine Energiequelle, die mit dem Energieverabreichungsabschnitt gekoppelt ist, der Gewebebehandlungsenergie überträgt, wobei die Energiequelle Ultraschallenergie aufweist; und
einen Prozessor, der mit dem Gewebeanalysator und der Energiequelle gekoppelt ist, wobei der Prozessor konfiguriert ist, eine geeignete Behandlungsenergie für das charakterisierte Körpergewebe durch Auswählen einer Energiewellenlänge der Ultraschallenergie beruhend auf der Charakterisierung des Körpergewebes so zu bestimmen, dass die Anwendung der geeigneten Behandlungsenergie das Körpergewebe mit dem Energieverabreichungsabschnitt sanft erwärmt, ohne das Körpergewebe zu ablatieren.

2. System nach Anspruch 1, wobei die Frequenz der Energie zwischen 1 kHz und 20 MHz liegt.

3. System nach Anspruch 1, wobei:

der Prozessor vorgegebene Behandlungsenergieeigenschaften aufweist, die zum sanften Erwärmen unterschiedlicher charakterisierter Materialien geeignet ist; und/oder
der Prozessor konfiguriert ist, die Behandlungsenergie als Reaktion auf eine Rückmeldung vom Gewebeanalysator während des Erwärmens des Körpergewebes einzustellen.

4. System nach Anspruch 1, wobei der Gewebeanalysator einen optischen Kohärenz-Tomographen aufweist, der mit mindestens einer optischen Leitung gekoppelt ist, die sich zwischen dem proximalen Ende des Katheters und mindestens einem radial ausgerichteten Fenster erstreckt, wobei der Tomograph konfiguriert ist, Bildsignale aus Abbildungslicht vom Körpergewebe zu erzeugen, um das Körpergewebe zu charakterisieren, wobei das Abbildungslicht durch das mindestens eine Fenster übertragen wird.

5. System nach Anspruch 1 zum sanften Erwärmen von Körpergewebe in der Nähe zu einer luminalen Oberfläche, die sowohl gesundes Gewebe als auch Zielgewebe aufweist, wobei der Prozessor konfiguriert ist, eine geeignete Behandlungsenergie zu bestimmen, das Körpergewebe ohne Ablatieren sanft auf eine Temperatur im Bereich von 45°C bis 65°C zu erwärmen.

6. System nach Anspruch 1, wobei der Prozessor konfiguriert ist, geeignete Parameter des fokussierten Ultraschalls für das charakterisierte Gewebe zu bestimmen, um das Gewebe ohne Ablatieren mit der geeigneten fokussierten Ultraschallenergie sanft auf eine Temperatur im Bereich von 45°C bis 65°C zu erwärmen.

7. System nach Anspruch 6 zum sanften Erwärmen von Körpergewebe in der Nähe zu einer luminalen Oberfläche, die sowohl gesundes Gewebe als auch Zielgewebe aufweist, auf eine Temperatur im Bereich von 45°C bis 65°C, die ausreicht, das Zielgewebe wirksam zu verändern, ohne eine übermäßige thermische Beschädigung am gesunden Gewebe zu verursachen.

**8.** System nach Anspruch 6, wobei der Gewebeanalysator eine Magnetresonanztomographievorrichtung ist.

**9.** System nach Anspruch 6, wobei der Prozessor konfiguriert ist, die fokussierte Ultraschallenergie als Reaktion auf eine Rückmeldung vom Gewebeanalysator während des Erwärmens des Gewebes einzustellen.

**10.** System nach Anspruch 6, wobei der Prozessor vorgegebene Eigenschaften der fokussierten Ultraschallenergie zum sanften Erwärmen unterschiedlicher charakterisierter Gewebe aufweist.

**Revendications**

**1.** Système de chauffage modéré d'un tissu corporel entourant un vaisseau sanguin, ledit système comprenant :

un cathéter oblong présentant une extrémité proximale et une extrémité distale, avec un axe s'étendant entre celles-ci ;
un ballonnet gonflable adjacent à l'extrémité distale du cathéter oblong ;
une partie d'administration énergétique prévue pour délivrer une énergie ultrasonore focalisée au tissu corporel, ladite partie d'administration énergétique comprenant au moins un transducteur ultrasonore monté sur une surface extérieure du ballonnet gonflable ;
un analyseur tissulaire prévu pour caractériser le tissu corporel dans le vaisseau sanguin à proximité de la partie d'administration énergétique ;
une source d'énergie reliée à la partie d'administration énergétique, transmettant une énergie de traitement tissulaire, ladite source d'énergie comprenant une énergie ultrasonore ; et
un processeur relié à l'analyseur tissulaire et à la source d'énergie, ledit processeur étant prévu pour déterminer une énergie de traitement adaptée au tissu corporel **caractérisé, par** sélection d'une longueur d'onde de l'énergie ultrasonore sur la base de la caractérisation du tissu corporel, de manière à obtenir un chauffage modéré du tissu corporel par application de l'énergie de traitement adaptée au moyen de la partie d'administration énergétique, sans ablation de tissu corporel.

**2.** Système selon la revendication 1, où la fréquence de l'énergie est comprise entre 1 kHz et 20 MHz.

**3.** Système selon la revendication 1, où :

le processeur présente des caractéristiques d'énergie de traitement définies, appropriées pour un chauffage modéré de différentes matières caractérisées ; et/ou
le processeur est prévu pour ajuster l'énergie de traitement en réponse à une information de retour de l'analyseur tissulaire pendant le chauffage du tissu corporel.

**4.** Système selon la revendication 1, où l'analyseur tissulaire comprend un tomographe à cohérence optique relié à au moins un guide optique s'étendant entre l'extrémité proximale du cathéter et au moins une fenêtre radialement orientée, ledit tomographe étant prévu pour générer des signaux d'image à partir d'une lumière d'imagerie du tissu corporel, de manière à caractériser le tissu corporel, la lumière d'imagerie étant transmises par la ou les fenêtres.

**5.** Système selon la revendication 1 pour le chauffage modéré d'un tissu corporel à proximité d'une surface luminale présentant un tissu sain ainsi qu'un tissu ciblé, le processeur étant prévu pour déterminer une énergie de traitement adaptée pour un chauffage modéré du tissu corporel à une température comprise entre 45 °C et 65 °C, sans ablation.

**6.** Système selon la revendication 1, où le processeur est prévu pour déterminer des paramètres d'ultrasons focalisés appropriés pour le tissu caractérisé, de manière à chauffer modérément le tissu avec l'énergie ultrasonore focalisée appropriée, à une température comprise entre 45 °C et 65 °C, sans ablation.

**7.** Système selon la revendication 6 pour le chauffage modéré d'un tissu corporel à proximité d'une surface luminale présentant un tissu sain ainsi qu'un tissu ciblé, à une température comprise entre 45 °C et 65 °C, suffisante pour altérer efficacement le tissu ciblé sans causer de dommage thermique excessif sur le tissu sain.

**8.** Système selon la revendication 6, où l'analyseur tissulaire est un dispositif d'imagerie à résonance magnétique.

**9.** Système selon la revendication 6, où le processeur est prévu pour ajuster l'énergie ultrasonore focalisée en réponse à une information de retour de l'analyseur tissulaire pendant le chauffage du tissu.

10. Système selon la revendication 6, où le processeur présente des caractéristiques définies pour l'énergie ultrasonore focalisée afin de chauffer modérément différents tissus caractérisés.

FIG. 1

FIG. 2

FIG. 3

FIG. 4

FIG. 5

FIG. 6

**FIG. 7**

**FIG. 8**

**FIG. 9**

FIG. 10A

FIG. 10B

FIG. 10C

**FIG. 11**

**FIG. 12A**

**FIG. 12B**

FIG. 13A

FIG. 13B

FIG. 14A

FIG. 14B

FIG. 15

FIG. 16

FIG. 17A    FIG. 17B    FIG. 17C    FIG. 17D

**EP 2 341 839 B1**

## REFERENCES CITED IN THE DESCRIPTION

**Patent documents cited in the description**

- US 6599288 B2
- US 20050251116 A